# EUROPEAN PATENT APPLICATION

(11) **EP 3 636 661 A1**
(43) Date of publication of application: **15.04.2020**
(21) Application number: 18200185.9
(22) Date of filing: 12.10.2018
(51) Int. Cl.: C07K 14/415, C12N 15/62, C12N 15/70, C12P 7/28, C12P 7/04

(54) **PHOSPHATE TRANSLOCATOR**

(71) Applicant: BIOC3, 31520 Ramonville-Saint-Agne (FR)
(72) Inventor: Cam, Yvan, 31320 Rebigue (FR)
(74) Representative: Bringer IP

(57) **Abstract**

The invention provides a polypeptide comprising a phosphate translocator and a microbial membrane-integrating protein. The phosphate translocator may be a plastidic phosphate translocator and the membrane-integrating protein may be derived from *Bacillus subtilis.* The invention also provides a bacterium genetically modified to export phosphorylated compounds; such organism may contain the polypeptide of the invention and may be further modified to decrease the metabolism of phosphorylated compounds or increase the production of phosphorylated compounds. Also provided is a method for the manufacture of phosphorylated compounds, comprising culturing a bacterium according to the invention and extracting the phosphorylated compounds from the culture medium. The method may be for the manufacture of dihydroxyacetone phosphate (DHAP), and optionally, may include the further step of converting the DHAP into methylglyoxal.

## Description

### Field of Invention

This invention relates to a polypeptide which can be used to export phosphorylated compounds from bacteria. The polypeptide comprises a phosphate translocator and a microbial membrane-integrating protein. The invention also relates to bacterial methods to produce and export phosphorylated products.

### Background to the Invention

High value chemical compounds are generally produced using chemical synthesis. However, these processes can be costly and result in low yields. Further, they have a significant environmental impact from the use of high temperature, high pressure and waste produced from organic solvents.

A number of compounds of interest are produced in nature by enzymes which are naturally found in organisms such as bacteria. As such, there has been much interest in recent years in harnessing bacterial metabolism to produce these desirable compounds. Methods have been developed, wherein microorganisms can be engineered to produce valuable chemicals as a sustainable and environmentally-friendly alternative to chemical synthesis.

One class of compounds which are highly desirable to produce are phosphorylated compounds, such as phosphorylated sugars. These compounds are very costly to produce, for example the current price of dihydroxyacetone phosphate (DHAP) is between €1200-6000 per gram. A number of phosphorylated sugars are produced naturally in many organisms, for example bacteria, through the process of glycolysis. However, these compounds are used in the bacterial metabolism and so do not generally accumulate within the bacteria and cannot be exported due to the presence of the negative charge on the bacterial membrane. There are also issues with engineering bacteria to produce excess of these compounds, for example if an excess of DHAP occurs this will degrade to methylglyoxal inside the cell which is toxic and results in cell death.

The present invention has developed a polypeptide that can be expressed in a bacterium to export these high value phosphorylated compounds across the bacterial membrane. Therefore, this has enabled the bacterial production of phosphorylated compounds. This approach will result in significant cost savings in the production of these products.

### Summary of the Invention

The present invention is based on the discovery that a bacterial cell can be genetically engineered to export phosphorylated compounds. As such these genetically engineered bacteria can be used to produce high value phosphorylated compounds in a more cost effective and environmentally friendly manner than traditional synthetic routes.

To allow the export of phosphorylated compounds an engineered polypeptide has been developed. This polypeptide comprises a phosphate translocator and a microbial membrane-integrating protein. Phosphate translocators are naturally found in plastids in plants, and are used to transport the products from photosynthesis out of the plastid for use in metabolic pathways. Surprisingly the combination of the translocator and microbial membrane-integrating protein allows expression of the phosphate translocator within a bacterial membrane, wherein it will recognise and export phosphorylated compounds.

This engineered polypeptide can be further combined with other genetic engineering approaches in order to enhance the production of phosphorylated compounds by the bacteria. For example, gene knock-outs can be produced which remove the genes necessary to produce enzymes which degrade the phosphorylated compounds. Using this approach a higher level of the phosphorylated products are produced by the bacteria and then exported.

Under normal circumstances the accumulation of phosphorylated compounds are toxic to the cell. In the present invention, due to the presence of the translocator these products are exported and so do not negatively impact the cell. Further, once the products are exported they can be easily extracted from the culture medium.

Therefore, one aspect of the invention relates to a polypeptide comprising a phosphate translocator and a microbial membrane-integrating protein.

A second aspect of the invention relates to a nucleic acid sequence which encodes the polypeptide comprising a phosphate translocator and a microbial membrane-integrating protein.

A third aspect of the invention relates to a vector comprising the nucleic acid sequence which encodes the polypeptide comprising a phosphate translocator and a microbial membrane-integrating protein.

A fourth aspect of the invention relates to bacterium genetically modified to export phosphorylated compounds.

A fifth aspect of the invention relates to a method for the manufacture of phosphorylated compounds, comprising the step of culturing a bacterium as defined above.

### Brief Description of the Figures

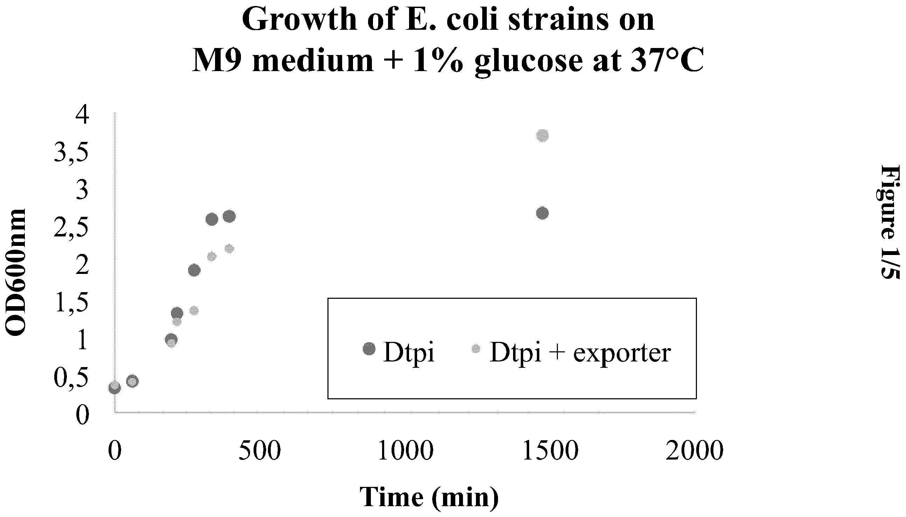

Figure 1 shows the optical density (OD) against time of two bacterial cultures. The first bacterial culture has been genetically engineered to knock-out the gene encoding triose phosphate isomerase, Dtpi (Δ*tpi*). The second bacterial culture has been genetically engineered to knock-out the gene encoding triose phosphate isomerase and transformed with a plasmid expressing a fusion protein of triose phosphate/phosphate translocator and a membrane-integrating protein known as Mistic, Dtpi + transporter. The OD is a measure of the cell growth.

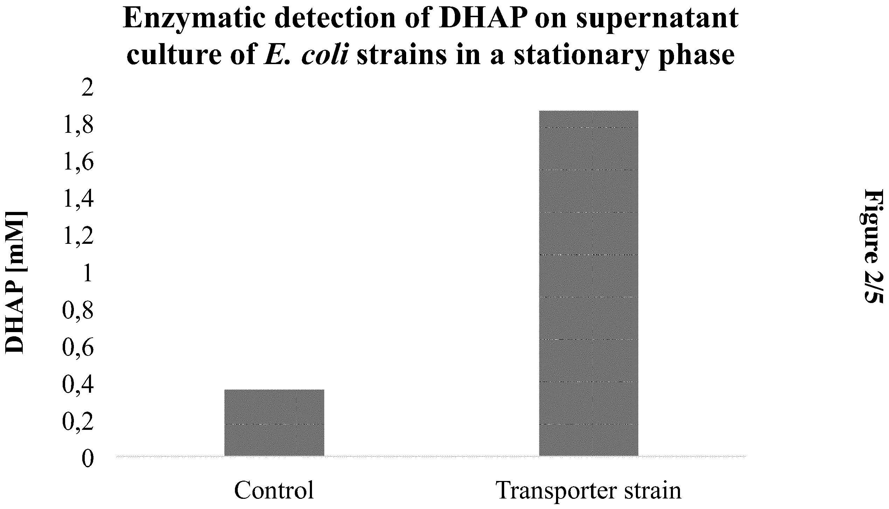

Figure 2 shows the concentration of DHAP detected in the supernatant of the bacterial cell culture of E.coli cells expressing the fusion protein of triose phosphate/phosphate translocator + Mistic and a comparative control culture which has not been genetically engineered. The concentration of DHAP was determined by an enzymatic assay described in example 3.

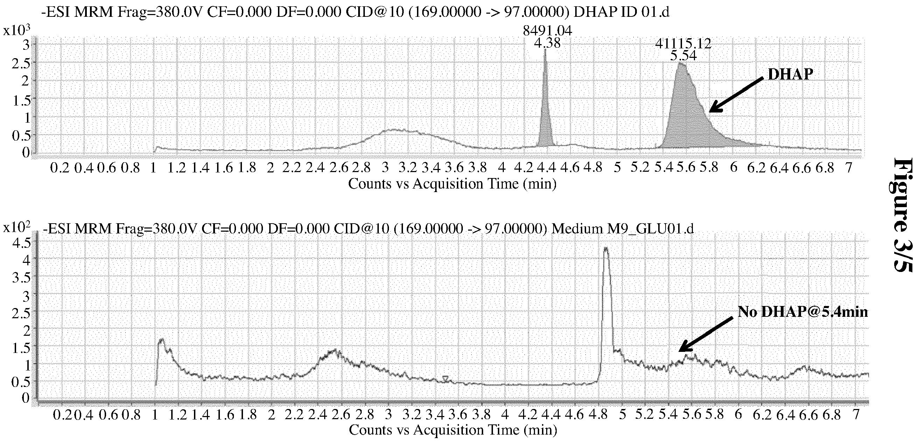

Figure 3 shows chromatograms of DHAP and media controls analysed using gas chromatography mass spectroscopy (GCMS). The upper panel shows the chromatogram of a control sample containing DHAP. A large peak can be seen with a counts v acquisition time of 5.54 min. The lower panel shows the chromatogram of a control sample of M9 medium. There is no peak present with a counts v acquisition time of approx. 5.54 min

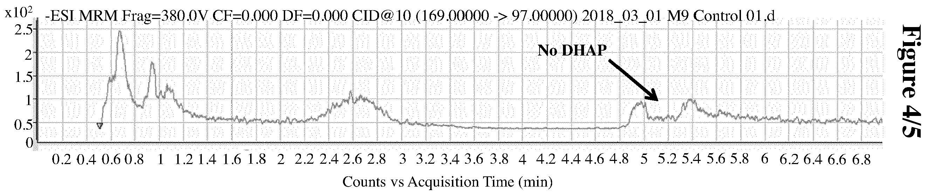

Figure 4 shows the chromatogram of M9 media in which a control *E.coli* strain has been grown. There is no peak at 5.4 min indicating that no DHAP is present in this sample.

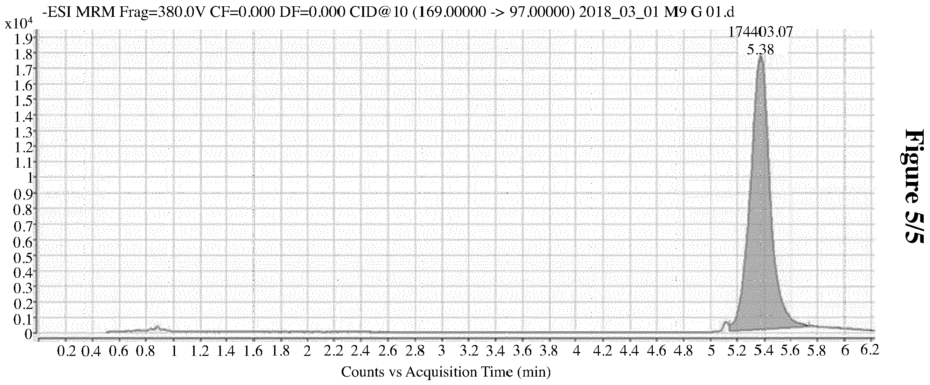

Figure 5 shows the chromatogram of M9 media in which an *E.coli* strain expressing the fusion protein of triose phosphate/phosphate translocator + Mistic has been grown. A large peak is seen at 5.38 min. This is in accordance with the counts v acquisition time of the DHAP control sample shown in the upper panel of Figure 3, therefore DHAP is present in this sample.

### Detailed Description of the Invention

In a first aspect of the present invention, there is provided a polypeptide comprising a phosphate translocator and a bacterial membrane-integrating protein.

As used herein the term "phosphate translocator" refers to a membrane protein which can transfer phosphorylated compounds across a membrane. These phosphate translocators are commonly found in plastids which are present in photosynthetic eukaryotes as well as other organisms which contain plastids. A plastid is an organelle that is surrounded by an inner and an outer membrane and has a number of different types of protein transporters in order the move compounds, such as proteins, amino acids, nucleic acids, peptides, sugars and phosphorylated sugars, in and out of the organelle. These translocators can be referred to as plastidic phosphate translocators and can be classified by their preferred substrate. Examples of plastidic phosphate translocators include triose-phosphate/phosphate translocator, glucose-6-phosphate/phosphate translocator, phosphoenol-pyruvate/phosphate translocator, or xylulose 5-phosphate/phosphate translocator.

The phosphate translocator, according to the first aspect of the invention, may be a plastidic phosphate translocator, wherein the phosphate translocator is naturally found expressed in the membrane of a plastid. Preferably the phosphate translocator is selected from the group comprising triose-phosphate/phosphate translocator, glucose-6-phosphate/phosphate translocator, phosphoenol-pyruvate/phosphate translocator, or xylulose 5-phosphate/phosphate translocator. In a preferred embodiment the phosphate translocator is a triose-phosphate/phosphate translocator. The triose-phosphate/phosphate translocator may have the amino acid sequence of SEQ ID NO 1.

As used herein the term "membrane integrating protein" refers to an integral membrane protein which can fold into a biological membrane. As used herein the term "membrane" refers to a lipid bilayer that acts as a boundary in an organism. A membrane integrating protein will be attached to the biological membrane. The protein may be polytopic wherein it spans the entire lipid bilayer of the biological membrane or it may be monotopic wherein it is attached to the membrane but does not span that entire lipid bilayer.

This membrane-integrating protein may be microbial; in particular it may be derived from bacteria, fungi, or archaea. Preferably the membrane integrating protein is derived from Bacilli bacteria, most preferably *Bacillis subtilis.* The membrane-integrating protein may fold autonomously into a membrane, in particular a bacterial membrane. By folding autonomously the membrane-integrating protein does not require a translocon in order to insert itself into the membrane.

A particularly preferred membrane-integrating protein is a protein known as Mistic. Mistic is an integral membrane protein that folds autonomously into a membrane and is conserved throughout Bacilli. According to an embodiment of invention the microbial membrane-integrating protein may comprise the amino acid sequence SEQ ID NO 2.

In order to link the phosphate translocator and microbial membrane-integrating protein they may be produced as a fusion protein by using a linker or spacer sequence. The linker sequence is a series of amino acid residues which separates the phosphate translocator and the membrane integrating protein. This series of amino acids may be chosen to provide a flexible linker sequence. Usually flexible linker sequences are glycine rich. The series of amino acids may be chosen to produce a rigid linker in or to prevent interaction of the two protein domains. Usually rigid linker sequences are proline rich and may form a coiled structure. In a highly preferred embodiment the linker sequence has the amino acid sequence SSSNNNNNLGSNGSG.

According to a second aspect of the invention there is provided a nucleotide sequence encoding a polypeptide as defined above. The nucleotide sequence may encode a phosphate translocator and a microbial membrane-integrating protein. Optionally the nucleotide sequence may also encode a suitable linker sequence to attach the phosphate translocator and a microbial membrane-integrating protein. In a highly preferred embodiment the nucleotide sequence comprises SEQ ID NO 4.

The person skilled in the art will appreciate that there are homologues, equivalents and derivatives of all of the nucleic acid and amino acid sequences described herein. Thus, the invention also encompasses nucleic acid molecules having a sequence substantially identical to the nucleic acid and amino acid sequences described herein over their entire length.

One of skill in the art will appreciate that the nucleic acid and amino acid sequences defined herein can also include variants of those particular nucleic acid molecules which are exemplified herein. These may occur in nature, for example because of strain variation. For example, additions, substitutions and/or deletions are included. One of skill in the art will also appreciate that variation from the particular nucleic acid molecules exemplified herein will be possible in view of the degeneracy of the genetic code. Preferably, the variants have substantial identity to the nucleic acid sequences described herein over their entire length.

As used herein, nucleic acid sequences which have "substantial identity" preferably have at least 80%, 90%, 91%, 92%, 93%, 94%, 95%, 96%, 97%, 98%, 98.1%, 98.2%, 98.3%, 98.4%, 98.5%, 98.6%, 98.7%, 98.8%, 98.9%, 99%, 99.1%, 99.2%, 99.3%, 99.4% 99.5%, 99.6%, 99.7%, 99.8% or 99.9% identity with said sequences. Desirably, the term "substantial identity" indicates that said sequence has a greater degree of identity with any of the sequences described herein than with prior art nucleic acid sequences.

The nucleotide sequence may be codon optimised for expression in a particular expression host such as bacteria. The nucleotide sequence may also comprise additional nucleotide sequences required for expression of the encoded polypeptide, such as promoter sequences, ribosome binding sites and nucleotide sequences for inducible expression of the protein. Suitable sequences will be known to those skilled in the art.

In order to express the encoded polypeptide the nucleotide sequence according to the second aspect of the invention, may be inserted into a suitable expression host. As such, a third aspect of the invention is vector comprising a nucleotide sequence as defined above.

A vector is a vehicle to deliver the nucleic acid sequence of interest into a suitable host to express the protein encoded by the nucleic acid sequence. Suitable vectors are known in the art, non-limiting examples of suitable vectors include plasmids, viral vectors, yeast artificial chromosomes and bacterial artificial chromosomes.

The vector can be transformed into a suitable host, such as a bacterial cell, a yeast cell, an insect cell or a mammalian cell. There are a variety of transformation, transfection and transduction methods that can be used in order to introduce the vector into a host cell. The skilled person will be able to determine the most appropriate method based on the type of vector used and the type of host cell.

According to a fourth aspect of the present invention, there is provided a bacterium genetically modified to export phosphorylated compounds. In particular the bacterium may be genetically modified to express a polypeptide as defined above. In order to express the polypeptide as defined above the bacterium may be transformed with a vector comprising a nucleic acid sequence which encodes a polypeptide comprising a phosphate translocator and a microbial membrane-integrating protein.

As used herein the terms "genetically modified" and "genetically engineered" are used interchangeably. As used herein these terms refer to the alteration of an organism's characteristics by altering the genetic material, which may be achieved by the introduction, deletion or modification of genetic material.

One class of phosphorylated compounds that are of particular interest are the phosphorylated sugars. In general a sugar may have the formula Cₙ(H₂O)ₘ and a phosphorylated sugar has an additional phosphoryl group attached. Non-limiting examples of phosphorylated sugars which can be exported by the genetically modified bacterium include dihydroxyacetone phosphate (DHAP) deoxyxylulose-5-phosphate, glucose-6-phosphate, fructose-6-phosphate, fructose-1,6-bisphosphate, glyceraldehyde-3-phosphate, glycerol-3-phosphate phosphoenolpyruvate, 2-phosphoglycerate, 3-phosphoglycerate, 1,3-diphosphoglycerate.

The bacterium may be genetically modified to express a membrane transporter, wherein the membrane transporter will transfer the phosphorylated compounds across the bacterial membrane. The transporter may export compounds via a number of different mechanisms for example diffusion, facilitated diffusion, primary active transport or secondary active transport. Examples of suitable membrane transporters include carrier proteins and channel proteins, α-type channels, β-barrel porins, uniporters, symporters, antiporters, phosphate translocators, nicotinamide ribonucleoside transporters.

In one embodiment the bacterium is genetically modified to express a phosphate translocator, preferably wherein the phosphate translocator is selected from the group comprising triose-phosphate/phosphate translocator, glucose-6-phosphate/phosphate translocator, phosphoenol-pyruvate/phosphate translocator, or xylulose 5-phosphate/phosphate translocator, more preferably wherein the phosphate translocator is a triose-phosphate/phosphate translocator.

In order to achieve export of the phosphorylated compounds it is preferred that the translocator or the fusion protein of the translocator and microbial membrane-integrating protein is constitutively expressed. As such the nucleic acid sequence encoding the translocator or the fusion protein of the translocator and microbial membrane-integrating protein may be under the control of a constitutive promoter such as galP promoter, T7 promoter, CMV promoter.

The phosphate translocator allows the export of phosphorylated compound produced by the bacterium. In order to enhance the amount of a phosphorylated compound of interest produced by a bacterium, further genetic engineering of the bacterium may be performed. The bacterium can be genetically modified in order to decrease the metabolism of phosphorylated compounds. By decreasing the metabolism of phosphorylated products this will increase the amount of phosphorylated products available to export. This can be achieved in a number of ways for example by inactivating a gene responsible for metabolising a compound of interest. Inactivating a gene may involve the complete or partial deletion of the gene of interest. There are multiple methods available for gene inactivation, which will be known to those skilled in the art. Non-limiting examples of techniques for gene inactivation include plasmid-mediated homologous recombination, linear DNA-mediated homologous recombination and insertional inactivation. The bacterium may also be genetically modified to increase the production of phosphorylated compounds. This can be achieved by overexpressing certain proteins in a metabolic pathway. Methods for the over expression of proteins are known within the art and include the use of inducible expression systems which may make use of inducible promotors, non-coding RNAs and riboswitches. These systems will enhance the expression of a protein encoded by a gene under the control of the inducible promotor, non-coding RNA or riboswitch.

In a preferred embodiment the bacterium is genetically engineered to export DHAP. In order to export DHAP the bacterium is genetically modified to express a triose-phosphate/phosphate translocator. The translocator is optionally produced as a fusion protein with a microbial membrane-integrating protein. In a preferred embodiment the bacterium expresses the amino acid sequence of SEQ ID NO 3. The expression of the triose-phosphate/phosphate translocator may be achieved by transforming the bacterium with a vector which encodes the translocator. The vector may comprise the nucleotide sequence of SEQ ID NO 4.

Optionally, genetic engineering can be performed to reduce triose phosphate isomerase activity. This may be achieved by inactivating the gene responsible for producing triose-phosphate isomerase, an enzyme which converts DHAP into glyceraldehyde-3-phosphate. Using this approach DHAP will not be utilised in the bacterial metabolism, therefore DHAP will begin to accumulate and so be exported via the triose-phosphate/phosphate translocator. In this embodiment, as the DHAP is not used by the bacterium, the DHAP that is produced will be exported and so a higher yield can be achieved.

A number of bacterial strains are suitable to be engineered to export phosphorylated compounds. These include gram negative bacteria for example Pseudomonas, Klebsiella, Enterobacter, Helicobacter, Salmonella. In a preferred embodiment *Escherichia coli* is genetically engineered.

According to a fifth aspect of the present invention there is provided a method for the manufacture of phosphorylated compounds, comprising the step of culturing a bacterium which has been genetically engineered as described above.

Bacterial culture conditions may depend on the strain of bacteria that is being used and can be optimised for the specific bacteria. A person skilled in the art will be able to optimise culture conditions for maximal protein expression or for maximal yield of the phosphorylated compound of interest. Suitable culture conditions include a culture temperature from about 20°C to about 45°C, preferably from about 30°C to about 37°C, most preferably about 37°C. Whilst the bacterial cells are cultured the containers may be agitated in order to allow aeration of the culture, agitation can be performed at about 100 rpm to about 250 rpm, preferably from about 180 rpm to about 220 rpm.

According to the fifth aspect of the invention, standard culture protocol may be used including first producing single colonies of the engineered bacterium of interest on solid agar plates, followed by production of a liquid media starter culture inoculated with a single colony, the starter culture can then be used to inoculate a larger volume of liquid media. The final culture volume may range from about 500 millilitres to about 2000 litres, preferably from about 500 millilitres to about 500 litres, more preferably from about 500 millilitres to 50 litres.

A number of different media are suitable for culturing the bacteria. As used herein the term "media" refers to a solid, liquid or semi-solid support in which microorganisms can be grown, for example a gel. The media can be supplemented with additional components in order to improve bacterial growth for example amino acids, sugars, lactose, glucose, mannose, glycerol, calcium, magnesium, iron, phosphate, sulphates, chlorides, acetates, citrates, etc. The media may also be supplemented with antibiotics or antimicrobials such as ampicillin, tetracycline, kanamycin, streptomycin, carbenicillin, erythromycin, and chloramphenicol. These antimicrobials may be used in order to selectively grow specific bacteria, wherein the bacteria contain a specific antibiotic resistance gene. Non-limiting examples of media which can be used to grow the bacteria include lysogeny broth (LB), terrific broth (TB), 2xYT broth, Super Optimal Broth (SOB), Super Optimal Broth with Catabolite repression (SOC), minimal salts media (M9), NZCYM broth, NZ amine broth.

According to the fifth aspect the bacterium may be a gram negative bacterium such as Pseudomonas, Klebsiella, Enterobacter, Helicobacter. In a preferred embodiment a genetically engineered *Escherichia coli* bacterium is used for the manufacture of phosphorylated compounds.

When the genetically engineered bacterium is cultured the phosphorylated compounds are exported through the bacterial membrane via the phosphate translocator. Therefore, the compounds of interest are present in the culture media. In an embodiment according to the fifth aspect of the present invention the method further comprises a step of extracting the phosphorylated compound from the culture media. The extraction process may be performed in various ways, however a key step is the separation of the bacterial cells from the liquid media which may be achieved by centrifugation, filtering or sedimentation. Once the liquid culture has been separated from the bacterial cells the compound of interest can then be purified using standard techniques such as column chromatography, high performance liquid chromatography, ion-exchange chromatography, thin-layer chromatography and reversed-phase chromatography.

The method according to the fifth aspect of the present invention can be used to manufacture a number of phosphorylated compounds. According to the fifth aspect the method may be used to manufacture phosphorylated sugars such as dihydroxyacetone phosphate (DHAP), deoxyxylulose-5-phosphate, glucose-6-phosphate, fructose-6-phosphate, fructose-1,6-bisphosphate, glyceraldehyde-3-phosphate, glycerol-3-phosphate phosphoenolpyruvate, 2-phosphoglycerate, 3-phosphoglycerate, 1,3-diphosphoglycerate. In a preferred embodiment the method is for the manufacture of DHAP.

The method can be used to manufacture a variety of high value phosphorylated compounds. These compounds can be used as a precursor for the synthesis of other high value compounds such a pharmaceuticals. In particular the method can be used to produce DHAP which can then be converted to methylglyoxal. In one embodiment the method according to the fifth aspect of the invention further comprises a step to convert DHAP into methylglyoxal. DHAP can be converted to methylglyoxal by thermal degradation. In order to convert DHAP into methylglyoxal the bacterial culture may be incubated at between approximately 25°C and approximately 45°C, more preferably between approximately 35°C and approximately 40°C, most preferably the bacterial culture is incubated at 37 °C. In order to convert DHAP into methylglyoxal, the DHAP may be first extracted and purified from the bacterial culture, or the DHAP may be converted to methylglyoxal before being extracted and purified from the bacterial culture.

The following examples illustrate the invention;

### Example 1 - Bacterial culture

Cells were cultured on lysogeny (LB) medium during genetic manipulations and during the first starter culture of cells that had been taken from the glycerol stock (30 % (v/v), kept at -80 °C) and plated on LB agar. LB agar was prepared adding 1.5 % of microbiological agar to the medium. Growth of the cultures was performed in M9 minimal medium that contained (D)-glucose at concentrations of 20 g/L together with 18 g/L Na₂HPO₄ · 12H₂O, 3 g/L KH₂PO₄, 0.5 g/L NaCl, 2/L g NH₄Cl, 0.5 g/L MgSO₄ · 7H₂O, 0.015 g/L CaCl₂ · 2H₂O, 0.010 g/L FeCl₃, 0.006 g/L Thiamine HCI, 1.2 mg/L MnSO₄ · H₂O, 1.2 mg/L CuCl₂ · 2H₂O. 3-(*N*-morpholino)propanesulfonic acid (MOPS) solution at pH 7 was used to buffer M9 minimal medium to a final concentration of 20 g/L after filter sterilization (Merck Millipore ExpressPlus). The media were filter sterilized or autoclaved. When required, antibiotics were added to the media at a concentration of 100 µg/mL of ampicillin. All chemicals were purchased from Sigma-Aldrich.

Starter cultures (10 mL of LB medium in 50 mL test tubes (BD Falcon)) were inoculated from frozen glycerol stocks and cultivated overnight. Cells from these cultures were used to inoculate 50 mL of glucose mineral medium in 250 mL shake flasks at OD -0.2. All cultures were carried out at 37 °C or 30°C on a rotary shaker (Infors HT) running at 200 rpm. Cell growth was followed by monitoring the OD600 using a spectrophotometer and culture supernatants were withdrawn for enzymatic test and HPLC analysis.

### Example 2 - Plasmid and strain construction

*Escherichia coli* K-12 substr. MG1655 was used as the parental strain throughout this study. The Δ*tpi* deletion mutants were created by the phage transduction method adapted from Miller [Miller et al., 1992]. The phage lysates were prepared from strains of the KEIO collection [Baba et al., 2006] which carried single deletions.

The plasmid carrying the triose phosphate/phosphate translocator was chemically synthetized by Genscript. The backbone is a pUC57 plasmid with an ampicillin resistant gene. The translocator gene, *tpt,* was codon optimized for *Escherichia coli and* a fusion was produced with a membrane-integrating protein known as Mistic which is found in *Bacillus subtilis.* The amino acid sequence for the fusion protein of the translocator and the Mistic protein is shown in SEQ ID NO 3 and the corresponding nucleotide sequence is shown in SEQ ID NO 4. A canonical ribosome binding site (RBS) and a constitutive promotor from *galP* were added to the sequence. Two restriction sites, Kpnl and Hindlll were added on 5' and 3' ends respectively.

Transformation was performed using standard protocols [Sambrook et al., 1989]. Chimio-competent cells were prepared using the one-step protocol from [Chung et al., 1989]. Transformed cells were selected on LB plates using 50 µg/ml of Ampicillin at 37°C overnight.

In order to determine the effect of the genetic engineering on the growth of the *E.coli* cells, growth assay in M9 minimal media were performed. The *E.coli* Δ*tpi* mutants were cultured according to the methods described in example 1 and the OD (optical density) of the culture was measured over time. An *E.coli* Δ*tpi* mutant transformed with the plasmid comprising the triose phosphate/phosphate translocator was also cultured under the same conditions and the OD measured over time. The results of this experiment are shown in Figure 1 and demonstrate that the combination of the Δ*tpi* and the expression of the translocator results in higher levels of growth as indicated by the higher OD value. It is probable that the increased level of growth is attainable due to the export of the DHAP. When DHAP is not exported and not degraded by triose phosphate isomerase, DHAP will convert into methylglyoxal which is toxic to the cells.
**SEQ ID NO 1 - amino acid sequence of triose phosphate/phosphate translocator (tpt) from *Arabidopsis thaliana.*** 
MESRVLLRATANVVGIPKLRRPIGAIHRQFSTASSSSFSVKPIGGIGEGANLISGRQLRP
ILLLDSSAINGGEKREILKPVKAAAAEGGDTAGDAKVGFLAKYPWLVTGFFFFMWYFLNV
IFNILNKKIYNYFPYPYFVSVIHLFVGVVYCLISWSVGLPKRAPIDSNLLKVLIPVAVCH
ALGHVTSNVSFAAVAVSFTHTIKALEPFFNAAASQFIMGQSIPITLWLSLAPVVLGVAMA
SLTELSFNWLGFISAMISNISFTYRSIFSKKAMTDMDSTNVYAYISIIALFVCIPPAIIV
EGPKLLNHGFADAIAKVGMTKFISDLFWVGMFYHLYNQLATNTLERVAPLTHAVGNVLKR
VFVIGFSIVIFGNKISTQTGIGTGIAIAGVAMYSIIKAKIEEEKRQGKKA
**SEQ ID NO 2 - amino acid sequence of Mistic from *Bacillus subtilis*** 
MKVTSDEKEQLSAAIDRMNEGLDVFIQFYNESEKDEPLIQLEDDTAKLMKEARELYGQDKLNETLNAIIKQILSISLSE
EGKKK
**SEQ ID NO 3 - amino acid sequence of fusion protein of Mistic and tpt, joined by a linker sequence which is underlined** 
MKVTSDEKEQLSAAIDRMNEGLDVFIQFYNESEKDEPLIQLEDDTAKLMKEARELYGQDKLNETLNAIIKQILSISLSEEGKKKSSSN
NNNNLGSNGSGMESRVLLRATANVVGIPKLRRPIGAIHRQFSTASSSSFSVKPIGGIGEGANLISGRQLRPILLLDSSAINGGEKREI
LKPVKAAAAEGGDTAGDAKVGFLAKYPWLVTGFFFFMWYFLNVIFNILNKKIYNYFPYPYFVSVIHLFVGVVYCLISWSVGLPKRA
PIDSNLLKVLIPVAVCHALGHVTSNVSFAAVAVSFTHTIKALEPFFNAAASQFIMGQSIPITLWLSLAPVVLGVAMASLTELSFNWL
GFISAMISNISFTYRSIFSKKAMTDMDSTNVYAYISIIALFVCIPPAIIVEGPKLLNHGFADAIAKVGMTKFISDLFWVGMFYHLYNQ
LATNTLERVAPLTHAVGNVLKRVFVIGFSIVIFGNKISTQTGIGTGIAIAGVAMYSIIKAKIEEEKRQGKKA
**SEQ ID NO 4 - *E. coli* codon optimised nucleotide sequence encoding the fusion protein of Mistic and tpt joined by a linker sequence**

ATGAAAGTTA CCTCCGACGA AAAAGAACAA TTATCGGCAG CCATCGACCG CATGAATGAG GGTCTTGACG
TTTTCATTCA GTTCTATAAC GAGAGTGAAA AAGATGAGCC GCTGATCCAA CTGGAGGATG ACACCGCTAA
ACTCATGAAG GAAGCACGTG AATTGTATGG CCAGGACAAA TTGAACGAGA CCCTGAACGC CATCATTAAA
CAAATTCTGA GTATTAGCCT GAGCGAAGAA GGAAAAAAAA AATCTTCTTC AAACAATAAC AACAACCTTG
GGTCAAATGG TAGCGGAATG GAGAGCCGTG TGCTGTTGCG TGCGACAGCT AATGTCGTCG GCATTCCGAA
ACTGCGTCGC CCGATCGGTG CCATCCACCG TCAATTTTCA ACGGCCAGCT CGAGCTCGTT TAGCGTTAAA
CCAATTGGCG GCATCGGCGA AGGCGCAAAC CTGATCAGCG GCCGCCAGTT ACGCCCGATT CTTCTGCTTG
ATTCTTCAGC AATCAATGGG GGCGAAAAGC GCGAAATTCT GAAGCCCGTA AAAGCGGCAG CGGCGGAAGG
CGGCGACACG GCTGGGGACG CGAAAGTCGG GTTTTTGGCG AAATATCCGT GGCTGGTCAC CGGCTTCTTT
TTTTTCATGT GGTATTTCCT TAACGTGATC TTTAACATTC TGAACAAAAA AATCTATAAT TACTTTCCGT ATCCGTACTT
CGTATCTGTG ATCCACCTCT TTGTAGGCGT TGTGTATTGT TTAATTAGCT GGAGCGTGGG CCTGCCGAAA
CGTGCTCCAA TTGACTCTAA CCTGCTGAAA GTCCTTATTC CGGTGGCGGT GTGCCATGCC CTCGGCCATG
TAACCAGCAA CGTTTCTTTC GCGGCAGTTG CCGTTTCGTT TACACACACT ATCAAAGCAT TGGAGCCATT TTTTAATGCC
GCGGCGTCGC AATTTATCAT GGGCCAGTCT ATCCCAATTA CCCTGTGGCT GAGTCTGGCA CCGGTCGTGC
TTGGTGTTGC TATGGCATCT CTGACAGAGC TGTCATTTAA CTGGCTGGGC TTTATTAGCG CCATGATTAG
CAATATCAGC TTTACGTACC GTTCAATCTT TTCCAAAAAA GCAATGACGG ACATGGATTC TACTAATGTG TATGCATACA
TTAGCATTAT CGCCTTATTC GTCTGCATCC CGCCAGCTAT CATTGTAGAG GGCCCGAAAT TGTTAAACCA TGGGTTTGCA
GATGCGATTG CAAAAGTGGG CATGACGAAA TTTATTTCAG ATCTGTTCTG GGTGGGTATG TTCTACCACC
TCTACAACCA GCTTGCGACT AACACTCTGG AACGCGTGGC GCCGCTGACC CATGCTGTGG GTAATGTGTT
GAAACGTGTT TTCGTCATTG GTTTCTCTAT CGTCATTTTC GGGAACAAAA TTAGTACCCA AACGGGTATT
GGCACTGGCA TCGCCATCGC AGGCGTTGCG ATGTACAGCA TTATCAAAGC AAAAATCGAA GAAGAAAAAC
GCCAAGGGAA AAAAGCA TAG

### Example 3 - Enzymatic assay

The supernatant from the bacterial culture was analysed using an enzymatic assay in order to determine the amount of DHAP exported into the media. DHAP concentrations were determined as follows. Triose phosphate isomerase (TPI) converts one molecule of DHAP into one molecule of Glyceraldehyde-3-phosphate (GAP). GAP reduction by GAP dehydrogenase (GAPdH) is then measured by following the formation of NADH. NADH absorbs at 340 nm. Reaction mixtures 250 µl contained 1 U TPI, 0.1 U GAPdH, 50 mM HEPES (pH 7.98), 1 mM dithiothreitol (DTT), 1mM NAD⁺, 2 mM Thiamine pyrophosphate and 200 µl of culture supernatant in 1 ml. The reaction takes place at 37°C during 10 minutes. The assay is performed at 22°C at 340 nm through a spectrophotometer.

Figure 2 demonstrates the results of the assay performed on culture media in which the Δ*tpi tpt* strain was cultured and media in which a control strain was cultured. The amount of DHAP detected in the culture media of the Δ*tpi tpt* strain is approximately 4.5 fold higher than the control.

### Example 4 - Analysis of phosphorylated products

To confirm the identity of the products being produced by the bacteria, structural analysis was performed. DHAP was analysed using an Agilent 1260 Infinity II LC system coupled to an Agilent 6490 triple quadrupole LC/MS system. Chromatography was performed using a Sequant ZIC HILIC (Merck, 100 x 2.1mm, 3.5µm film thickness, 100A°) under isothermal conditions (35 °C) and using a binary gradient. Solvent A was 10 mM aqueous ammonium bicarbonate pH 9.0 (pH was adjusted using ammonium hydroxide solution) and solvent B was acetonitrile. The injection volume of filtered samples (through 0.45 µm filters) was 5 µl and a flow-rate of 0.5 ml/min was applied. Gradients applied for separation and the triple quadrupole detector conditions were presented in table I and table II. Instrument control, data acquisition, processing, and report generation were accomplished through the Agilent MassHunter Workstation Data Acquisition. All stock standard solutions for individual analytes were prepared in deionized water (D.I. water, 18.2 MΩ-cm-1, Millipore)-acetonitrile solution. Standard for injection has an approximate concentration of 0.1 ng/mL.

**Table 1 - HPLC gradient and parameters**

| **Time (min)** | **Mobile phase A (%)** | **Mobile phase B (%)** |
|---|---|---|
| Initial | 2 | 98 |
| 6 | 30 | 70 |
| 6.10 | 0 | 100 |
| 8 | 0 | 100 |

**Table 2. Triple quadrupole detector conditions in MRM operating mode**

| **Compound name** | **Prec lon (m/z)** | **Prod ion (m/z)** | **CE (V)** | **Cell acc. (V)** | **Polarity** |
|---|---|---|---|---|---|
| DHAP | 169 | 97 | 10 | 5 | Negative |

## Claims

1. A polypeptide comprising a phosphate translocator and a microbial membrane-integrating protein.

2. The polypeptide of claim 1, wherein the phosphate translocator is a plastidic phosphate translocator, preferably wherein the phosphate translocator is selected from the group comprising triose-phosphate/phosphate translocator, glucose-6-phosphate/phosphate translocator, phosphoenol-pyruvate/phosphate translocator, or xylulose 5-phosphate/phosphate translocator, preferably wherein the phosphate translocator is a triose-phosphate/phosphate translocator.

3. The polypeptide of claim 1 or 2, wherein the membrane-integrating protein is derived from *Bacillus subtilis,* preferably wherein the bacterial membrane-integrating protein comprises the amino acid sequence SEQ ID NO 2.

4. A nucleotide sequence encoding a polypeptide according to any of claims 1 to 3.

5. A vector comprising a nucleotide sequence according to claim 4.

6. The vector according to claim 5, wherein the vector is a plasmid or a viral vector.

7. A bacterium genetically modified to export phosphorylated compounds.

8. The bacterium of claim 7, wherein the bacterium is genetically modified to express a membrane transporter, preferably a phosphate translocator, more preferably wherein the phosphate translocator is selected from the group comprising triose-phosphate/phosphate translocator, glucose-6-phosphate/phosphate translocator, phosphoenol-pyruvate/phosphate translocator, or xylulose 5-phosphate/phosphate translocator, most preferably wherein the phosphate translocator is a triose-phosphate/phosphate translocator.

9. The bacterium of claim 7 or 8, wherein the bacterium is genetically modified to express a polypeptide according to any of claims 1 to 3.

10. The bacterium of any of claims 7 to 9, wherein the bacterium is further genetically modified to decrease the metabolism of phosphorylated compounds or to increase the production of phosphorylated compounds.

11. The bacterium of any of claims 7 to 10, wherein the bacterium is further genetically modified to delete the gene encoding triose-phosphate isomerase, or to remove triose-phosphate isomerase activity.

12. The bacterium of any of claims 7 to 11, wherein the bacterium is gram negative, preferably wherein the bacterium is *Escherichia coli.*

13. A method for the manufacture of phosphorylated compounds, comprising the step of culturing a bacterium according to any of claims 7 to 12.

14. The method according to claim 13, further comprising the step of extracting the phosphorylated compounds from the culture medium.

15. The method of claim 13 or 14, wherein the method is for the manufacture of dihydroxyacetone phosphate (DHAP), and optionally, wherein the method includes a further step to convert the DHAP into methylglyoxal and/or D-glyceraldehyde.


SEQUENCE LISTING

<110>  Biotech Fine Chemical France

<120>  POLYPEPTIDE

<130>  RMC00130EP

<160>  4

<170>  PatentIn version 3.5

<210>  1
<211>  410
<212>  PRT
<213>  Arabidopsis thaliana

<400>  1

Met Glu Ser Arg Val Leu Leu Arg Ala Thr Ala Asn Val Val Gly Ile
1               5                   10                  15

Pro Lys Leu Arg Arg Pro Ile Gly Ala Ile His Arg Gln Phe Ser Thr
            20                  25                  30

Ala Ser Ser Ser Ser Phe Ser Val Lys Pro Ile Gly Gly Ile Gly Glu
        35                  40                  45

Gly Ala Asn Leu Ile Ser Gly Arg Gln Leu Arg Pro Ile Leu Leu Leu
    50                  55                  60

Asp Ser Ser Ala Ile Asn Gly Gly Glu Lys Arg Glu Ile Leu Lys Pro
65                  70                  75                  80

Val Lys Ala Ala Ala Ala Glu Gly Gly Asp Thr Ala Gly Asp Ala Lys
                85                  90                  95

Val Gly Phe Leu Ala Lys Tyr Pro Trp Leu Val Thr Gly Phe Phe Phe
            100                 105                 110

Phe Met Trp Tyr Phe Leu Asn Val Ile Phe Asn Ile Leu Asn Lys Lys
        115                 120                 125

Ile Tyr Asn Tyr Phe Pro Tyr Pro Tyr Phe Val Ser Val Ile His Leu
    130                 135                 140

Phe Val Gly Val Val Tyr Cys Leu Ile Ser Trp Ser Val Gly Leu Pro
145                 150                 155                 160

Lys Arg Ala Pro Ile Asp Ser Asn Leu Leu Lys Val Leu Ile Pro Val
                165                 170                 175

Ala Val Cys His Ala Leu Gly His Val Thr Ser Asn Val Ser Phe Ala
            180                 185                 190

Ala Val Ala Val Ser Phe Thr His Thr Ile Lys Ala Leu Glu Pro Phe
        195                 200                 205

Phe Asn Ala Ala Ala Ser Gln Phe Ile Met Gly Gln Ser Ile Pro Ile
    210                 215                 220

Thr Leu Trp Leu Ser Leu Ala Pro Val Val Leu Gly Val Ala Met Ala
225                 230                 235                 240

Ser Leu Thr Glu Leu Ser Phe Asn Trp Leu Gly Phe Ile Ser Ala Met
                245                 250                 255

Ile Ser Asn Ile Ser Phe Thr Tyr Arg Ser Ile Phe Ser Lys Lys Ala
            260                 265                 270

Met Thr Asp Met Asp Ser Thr Asn Val Tyr Ala Tyr Ile Ser Ile Ile
        275                 280                 285

Ala Leu Phe Val Cys Ile Pro Pro Ala Ile Ile Val Glu Gly Pro Lys
    290                 295                 300

Leu Leu Asn His Gly Phe Ala Asp Ala Ile Ala Lys Val Gly Met Thr
305                 310                 315                 320

Lys Phe Ile Ser Asp Leu Phe Trp Val Gly Met Phe Tyr His Leu Tyr
                325                 330                 335

Asn Gln Leu Ala Thr Asn Thr Leu Glu Arg Val Ala Pro Leu Thr His
            340                 345                 350

Ala Val Gly Asn Val Leu Lys Arg Val Phe Val Ile Gly Phe Ser Ile
        355                 360                 365

Val Ile Phe Gly Asn Lys Ile Ser Thr Gln Thr Gly Ile Gly Thr Gly
    370                 375                 380

Ile Ala Ile Ala Gly Val Ala Met Tyr Ser Ile Ile Lys Ala Lys Ile
385                 390                 395                 400

Glu Glu Glu Lys Arg Gln Gly Lys Lys Ala
                405                 410

<210>  2
<211>  84
<212>  PRT

<213>  Bacillus subtilis

<400>  2

Met Lys Val Thr Ser Asp Glu Lys Glu Gln Leu Ser Ala Ala Ile Asp
1               5                   10                  15

Arg Met Asn Glu Gly Leu Asp Val Phe Ile Gln Phe Tyr Asn Glu Ser
            20                  25                  30

Glu Lys Asp Glu Pro Leu Ile Gln Leu Glu Asp Asp Thr Ala Lys Leu
        35                  40                  45

Met Lys Glu Ala Arg Glu Leu Tyr Gly Gln Asp Lys Leu Asn Glu Thr
    50                  55                  60

Leu Asn Ala Ile Ile Lys Gln Ile Leu Ser Ile Ser Leu Ser Glu Glu
65                  70                  75                  80

Gly Lys Lys Lys

<210>  3
<211>  509
<212>  PRT
<213>  synthetic

<400>  3

Met Lys Val Thr Ser Asp Glu Lys Glu Gln Leu Ser Ala Ala Ile Asp
1               5                   10                  15

Arg Met Asn Glu Gly Leu Asp Val Phe Ile Gln Phe Tyr Asn Glu Ser
            20                  25                  30

Glu Lys Asp Glu Pro Leu Ile Gln Leu Glu Asp Asp Thr Ala Lys Leu
        35                  40                  45

Met Lys Glu Ala Arg Glu Leu Tyr Gly Gln Asp Lys Leu Asn Glu Thr
    50                  55                  60

Leu Asn Ala Ile Ile Lys Gln Ile Leu Ser Ile Ser Leu Ser Glu Glu
65                  70                  75                  80

Gly Lys Lys Lys Ser Ser Ser Asn Asn Asn Asn Asn Leu Gly Ser Asn
                85                  90                  95

Gly Ser Gly Met Glu Ser Arg Val Leu Leu Arg Ala Thr Ala Asn Val
            100                 105                 110

Val Gly Ile Pro Lys Leu Arg Arg Pro Ile Gly Ala Ile His Arg Gln
        115                 120                 125

Phe Ser Thr Ala Ser Ser Ser Ser Phe Ser Val Lys Pro Ile Gly Gly
    130                 135                 140

Ile Gly Glu Gly Ala Asn Leu Ile Ser Gly Arg Gln Leu Arg Pro Ile
145                 150                 155                 160

Leu Leu Leu Asp Ser Ser Ala Ile Asn Gly Gly Glu Lys Arg Glu Ile
                165                 170                 175

Leu Lys Pro Val Lys Ala Ala Ala Ala Glu Gly Gly Asp Thr Ala Gly
            180                 185                 190

Asp Ala Lys Val Gly Phe Leu Ala Lys Tyr Pro Trp Leu Val Thr Gly
        195                 200                 205

Phe Phe Phe Phe Met Trp Tyr Phe Leu Asn Val Ile Phe Asn Ile Leu
    210                 215                 220

Asn Lys Lys Ile Tyr Asn Tyr Phe Pro Tyr Pro Tyr Phe Val Ser Val
225                 230                 235                 240

Ile His Leu Phe Val Gly Val Val Tyr Cys Leu Ile Ser Trp Ser Val
                245                 250                 255

Gly Leu Pro Lys Arg Ala Pro Ile Asp Ser Asn Leu Leu Lys Val Leu
            260                 265                 270

Ile Pro Val Ala Val Cys His Ala Leu Gly His Val Thr Ser Asn Val
        275                 280                 285

Ser Phe Ala Ala Val Ala Val Ser Phe Thr His Thr Ile Lys Ala Leu
    290                 295                 300

Glu Pro Phe Phe Asn Ala Ala Ala Ser Gln Phe Ile Met Gly Gln Ser
305                 310                 315                 320

Ile Pro Ile Thr Leu Trp Leu Ser Leu Ala Pro Val Val Leu Gly Val
                325                 330                 335

Ala Met Ala Ser Leu Thr Glu Leu Ser Phe Asn Trp Leu Gly Phe Ile
            340                 345                 350

Ser Ala Met Ile Ser Asn Ile Ser Phe Thr Tyr Arg Ser Ile Phe Ser
        355                 360                 365

Lys Lys Ala Met Thr Asp Met Asp Ser Thr Asn Val Tyr Ala Tyr Ile
    370                 375                 380

Ser Ile Ile Ala Leu Phe Val Cys Ile Pro Pro Ala Ile Ile Val Glu
385                 390                 395                 400

Gly Pro Lys Leu Leu Asn His Gly Phe Ala Asp Ala Ile Ala Lys Val
                405                 410                 415

Gly Met Thr Lys Phe Ile Ser Asp Leu Phe Trp Val Gly Met Phe Tyr
            420                 425                 430

His Leu Tyr Asn Gln Leu Ala Thr Asn Thr Leu Glu Arg Val Ala Pro
        435                 440                 445

Leu Thr His Ala Val Gly Asn Val Leu Lys Arg Val Phe Val Ile Gly
    450                 455                 460

Phe Ser Ile Val Ile Phe Gly Asn Lys Ile Ser Thr Gln Thr Gly Ile
465                 470                 475                 480

Gly Thr Gly Ile Ala Ile Ala Gly Val Ala Met Tyr Ser Ile Ile Lys
                485                 490                 495

Ala Lys Ile Glu Glu Glu Lys Arg Gln Gly Lys Lys Ala
            500                 505

<210>  4
<211>  1530
<212>  DNA
<213>  synthetic

<400>  4
atgaaagtta cctccgacga aaaagaacaa ttatcggcag ccatcgaccg catgaatgag      60

ggtcttgacg ttttcattca gttctataac gagagtgaaa aagatgagcc gctgatccaa     120

ctggaggatg acaccgctaa actcatgaag gaagcacgtg aattgtatgg ccaggacaaa     180

ttgaacgaga ccctgaacgc catcattaaa caaattctga gtattagcct gagcgaagaa     240

ggaaaaaaaa aatcttcttc aaacaataac aacaaccttg ggtcaaatgg tagcggaatg     300

gagagccgtg tgctgttgcg tgcgacagct aatgtcgtcg gcattccgaa actgcgtcgc     360

ccgatcggtg ccatccaccg tcaattttca acggccagct cgagctcgtt tagcgttaaa     420

ccaattggcg gcatcggcga aggcgcaaac ctgatcagcg gccgccagtt acgcccgatt     480

cttctgcttg attcttcagc aatcaatggg ggcgaaaagc gcgaaattct gaagcccgta     540

aaagcggcag cggcggaagg cggcgacacg gctggggacg cgaaagtcgg gtttttggcg     600

aaatatccgt ggctggtcac cggcttcttt tttttcatgt ggtatttcct taacgtgatc 660

tttaacattc tgaacaaaaa aatctataat tactttccgt atccgtactt cgtatctgtg 720

atccacctct ttgtaggcgt tgtgtattgt ttaattagct ggagcgtggg cctgccgaaa 780

cgtgctccaa ttgactctaa cctgctgaaa gtccttattc cggtggcggt gtgccatgcc 840

ctcggccatg taaccagcaa cgtttctttc gcggcagttg ccgtttcgtt tacacacact 900

atcaaagcat tggagccatt ttttaatgcc gcggcgtcgc aatttatcat gggccagtct 960

atcccaatta ccctgtggct gagtctggca ccggtcgtgc ttggtgttgc tatggcatct 1020

ctgacagagc tgtcatttaa ctggctgggc tttattagcg ccatgattag caatatcagc 1080

tttacgtacc gttcaatctt ttccaaaaaa gcaatgacgg acatggattc tactaatgtg 1140

tatgcataca ttagcattat cgccttattc gtctgcatcc cgccagctat cattgtagag 1200

ggcccgaaat tgttaaacca tgggtttgca gatgcgattg caaaagtggg catgacgaaa 1260

tttatttcag atctgttctg ggtgggtatg ttctaccacc tctacaacca gcttgcgact 1320

aacactctgg aacgcgtggc gccgctgacc catgctgtgg gtaatgtgtt gaaacgtgtt 1380

ttcgtcattg gtttctctat cgtcattttc gggaacaaaa ttagtaccca aacgggtatt 1440

ggcactggca tcgccatcgc aggcgttgcg atgtacagca ttatcaaagc aaaaatcgaa 1500

gaagaaaaac gccaagggaa aaaagcatag 1530